# EUROPÄISCHE PATENTSCHRIFT

(11) **EP 4 426 241 B1**
(45) Veröffentlichungstag und Bekanntmachung des Hinweises auf die Patenterteilung: **08.10.2025**
(21) Anmeldenummer: 22813907.7
(22) Anmeldetag: 02.11.2022
(51) Int. Cl.: A61F 5/01

(54) **DYNAMISCH ABHÄNGIGES BEWEGUNGSBLOCKADESYSTEMS**
DYNAMICALLY DEPENDENT MOVEMENT BLOCKING SYSTEM
SYSTÈME DE BLOCAGE DE MOUVEMENT DYNAMIQUEMENT DÉPENDANT

(30) Priorität: 02.11.2021 AT 602722021
(43) Veröffentlichungstag der Anmeldung: 11.09.2024
(73) Patentinhaber: O'NEAL Europe GmbH & Co. KG, 71665 Vaihingen an der Enz (DE)
(72) Erfinder: SAIER, Thomas, 9020 Klagenfurt (AT); RAFOLT, Dietmar, 8043 Graz (AT); SCHRECKENSBERGER, Peter, 4690 Schwanenstadt (AT); RUSS, David, 8508 St. Nololai im Sausal (AT); FALK, Eduard, 8010 Graz (AT)
(74) Vertreter: Meissner Bolte Partnerschaft mbB
(86) Internationale Anmeldenummer: PCT/EP2022/080498
(87) Internationale Veröffentlichungsnummer: WO 2023/078893

(56) Entgegenhaltungen:
- DE-B3- 102018 116 569
- US-A- 5 712 011
- US-A1- 2021 030 575

## Beschreibung

### Hintergrund der Erfindung:

Aufgrund der bestehenden PSA Verordnung (persönliche Schutzausrüstung) CEN Norm 1621, sind bestehende Körperprotektoren nur auf direkte Schläge gegen den Körper ausgelegt. Aus medizinischer Sicht bestätigt sich, dass die überwiegende Mehrzahl von Körperverletzungen auf Überschreitungen der natürlichen physiologischen Bewegungsbereiche des menschlichen Körpers zurückzuführen sind.

In der vorliegenden Patenteinreichung werden Strukturen beschrieben, die in den jeweiligen Körperregionen physiologische Überschreitungen verhindern können.

Diese Strukturen setzen sich aus:
- Anbindungsstrukturen an den Gelenkssegmenten, sowie
- Verbindungsstrukturen zwischen den jeweiligen Anbindungsstrukturen zusammen.

US 2021/030575 A1 offenbart ein Gerät zur Begrenzung der physiologischen Bewegungen von Körpersegmenten, wobei Körpersegmente mittels eines Auszugelementes und eines Blockadeelementes miteinander verbunden sind. Das Blockadeelement beinhaltet ein in einer Kammer befindliches, scherverdickendes Medium, das zu einer Dämpfung von Bewegungen der mit dem Auszugelement verbundenen Körpersegmenten beiträgt. Auch US 5 712 011 A offenbart einen Mechanismus zur Dämpfung von Körperbewegungen, der auf der Verwendung eines scherverdickenden Mediums beruht. Eine weitere Lösung zur Dämpfung von Körperbewegungen, die auf der Dämpfungswirkung eines Füllmediums beruht, ist aus der DE 10 2018 116569 B3 bekannt.

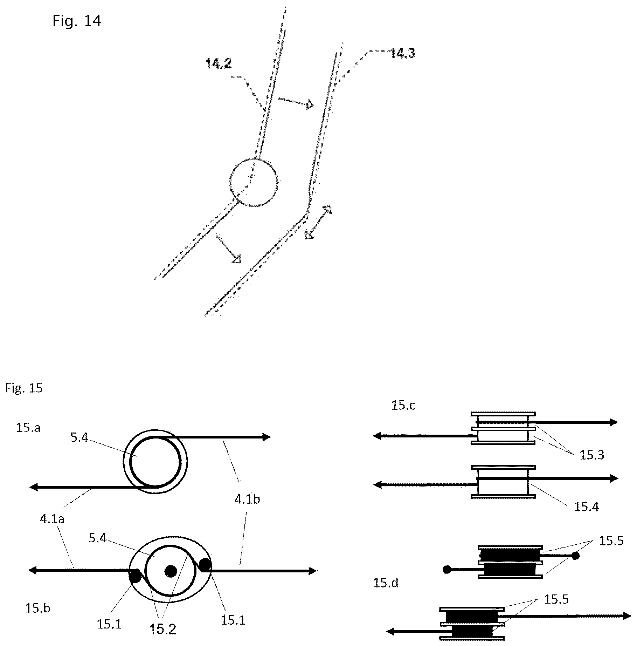

Bei der Definition des Wirkungsgrades von Verbindungsstrukturen am menschlichen Körper muss berücksichtigt werden, dass neben harten knöchernen Strukturen auch elastisches Gewebe wie Knorpel, Muskel, Bindegewebe, Fett etc. bei der Übertragung von Kräften berücksichtigt werden müssen. Je nach der Führung von Kraftlinien bei der Verwendung von Verbindungsstrukturen die an den jeweiligen Anbindungsstrukturen angebracht sind, kommen diese Elastizitäten und Kompressibilitäten zum Tragen. Dies kann auch durch zusätzlich dynamischen Prozessen verstärkt werden. Dies tritt beispielsweise auf, wenn Kraftlinien an der Oberfläche des menschlichen Körpers abseits der neutralen Biegelinie (Fig. 14) (14.2) geführt werden. Eine Steigerung dieser Problematik liegt vor, wenn Gelenkssegmente kaskadiert sind, wie beispielsweise bei der Wirbelsäule.

Das zeigt sich in der Protektion im Sport und der Arbeitswelt und besonders in der Rehabilitation, da eine Bewegungsbeschränkung durch Blockade der Segmente schon vor Eintreten der Maximalauslenkung der Körpersegmente eingeleitet werden müssen, um vor Verletzungen zu schützen.

Der Erfindung liegt daher die Aufgabe zugrunde, ein Blockadesystem gemäß Patentanspruch 1. darzustellen, welches in einer Verbindungsstruktur integriert ist, abhängig von Geschwindigkeit und Auslenkungswinkel die natürliche Bewegung der Körpersegmente zulässt und ab einen Geschwindigkeitsschwellwert eine Blockade einleitet.

### Stand der Technik:

Im Zuge der Recherche wurde folgender Stand der Technik erhoben:
EP2854720B1; EP3145455B1; US10 098775B2; EP3294236B1; US 20170304057A1;DE 102017117786B4; DE102018116569B3; DE 102016114110A1; DE 102017109877A1; EP0368798B1; EP0682483B1; US4741115; US6202953B1; US7591050B2; US8277401B2; USD663850S; USD666301S; USD666302S; USD 758061S; US8597369B2; US5762599A; US6033334A; US8968227B2; US20120209405A1; US7811333B2; US8696764B2; US20100032239A1; US5165510A; US20110031800A1

### Im Gegensatz zu Patent:

EP2854720B1; EP3145455B1; US10 098775B2; EP3294236B1; US 20170304057A1;DE 102017117786B4; DE102018116569B3; DE 102016114110A1; DE 102017109877A1
grenzt sich die vorliegende Erfindung dadurch ab, dass vorwiegend mechanische und / oder elektronische Arretierungsmechanismen angewendet werden. Die Eigenschaften von viskosen Flüssigkeiten sind temperaturabhängig. Somit ist es schwer realisierbar dieselben Auslöseeigenschaften in einem Temperaturbereich von beispielsweise -40° Celsius bis hin zu +50° Celsius mit Mechanismen zu realisieren, die durch die Eigenschaften von viskosen Flüssigkeiten abhängig sind. Der Vorteil einer mechanischen Arretierung (vorliegende Erfindung) liegt darin, dass Temperaturschwankungen nur sehr geringen Einfluss auf die Blockadeeigenschaften des Bauteils haben.

### Im Gegensatz zu Patent:

EP0368798B1; EP0682483B1; US4741115; US6202953B1; US7591050B2;
US8277401B2; USD663850S; USD666301S; USD666302S; USD 758061S;
grenzt sich die vorliegende Erfindung dadurch ab, dass das System nicht zur Schnürung von gegenüberliegenden Kleidungsstücken besteht. Die vorliegende Erfindung funktioniert nach demselben Prinzip wie ein Sicherheitsgurt. Die erfindungsgemäße Vorrichtung ist unter einem definierten Geschwindigkeitsschwellwert beweglich und arretiert erst oberhalb dieses Schwellwertes mit Hilfe eines Blockademechanismus.

Das Grundprinzip der vorliegende Erfindung besteht darin, dass eine Rotor (5.4), auf dem ein Auszugselement (1.4) in Form eines Seiles, Gurtes etc. aufgewickelt ist, durch einen Blockademechanismus (1.1.3) ab einer bestimmten Drehzahl blockiert und ein weiteres Ausziehen des Auszugelementes (1.4) verhindert, wodurch die beiden Körpersegmente, die mit dem Blockadesystem (1.1) miteinander verbunden sind, sich nicht weiter voneinander entfernen können. Das wäre beispielsweise eine Streckung des Ellenbogens, wenn Ober- und Unterarm quasi parallel zum Bizeps verbunden sind, oder eine Rotation des Oberkörpers, wenn die Schulter- und Hüftregion diagonal mittels des Blockademechanismus miteinander verbunden werden.

Die Blockade erfolgt mittels eines Kontaktelementes (5.6), der infolge Fliehkraft nach außen gedrängt wird. Damit die Fliehkraft das Kontaktelement (5.6) bewegt, muss dessen Drehpunkt außerhalb des Masseschwerpunktes liegen. Ab einer bestimmten Auslenkung hackt das Kontaktelement (5.6) in einer Formschlussstruktur (5.3) am Stator (Gehäuse) formschlüssig ein und die Rotation wird gestoppt. Die zentrifugale Bewegung resultiert aus der Fliehkraft und der Kraft einer Rückholblattfeder (5.7), die das Kontaktelement (5.6) bei niedriger Drehzahl wieder nach innen zieht. Die Steifigkeit dieser Feder bestimmt die Auslösedrehzahl und kann variiert werden.

Das Blockadesystem (1.1) besteht somit aus zwei Ebenen. In der ersten körpernahen Ebene befindet sich der Blockademechanismus (1.1.3), in der zweiten Ebene der Aufspulmechanismus (1.1.2), wobei die Reihenfolge beliebig ist.

Mit derselben Anordnung, jedoch ohne formschlüssiges Profil in der Formschlussstruktur (5.3) kann eine Blockade in Form einer Rutschkupplung (ähnlich wie Trommelbremse) realisiert werden. Je höher die Drehzahl umso höher ist die Bremswirkung, die bis zur Relativbewegung 0 gehen kann.

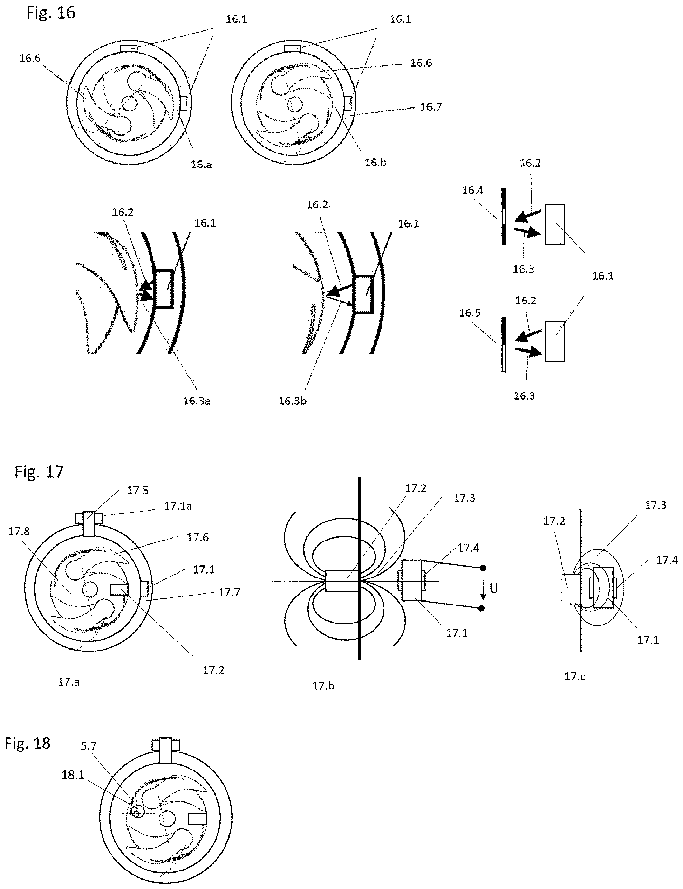

Dieses selbstauslösendes System bezieht die Energie zur Aktivierung der Blockade ausschließlich vom Auszugmechanismus selbst, also von der Bewegung der Körpersegmente. Die Masse des Kontaktelementes (5.6) sowie die Federkraft der Rückholfeder (5.7) sind konstant und demzufolge auch die Auszugsgeschwindigkeit des Auszugelementes (1.4), die die Blockade einleitet. Durch Variation dieser beiden Komponenten kann das Verhalten definiert werden. Dazu kann eine exzentrische Stellschraube (18.1) eingesetzt werden, die die Vorspannung der Blattfeder variiert (Fig.18).

### Gesteuertes Blockadesystem mit Hilfsenergie:

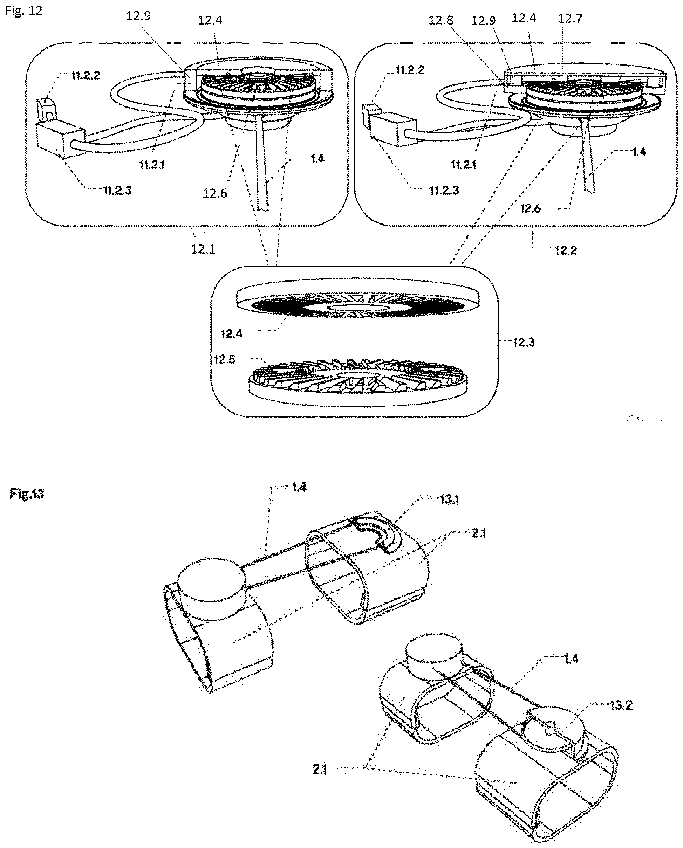

Eine weitere neue, hier vorgestellte Vorrichtung bezieht die Energie zur Blockade von einem elektromagnetischen System. Da heißt, es wird kein Fliehkraft benötigt, sondern der rotierende Teil und der stehende Teil besitzt je eine gegenseitige formschlüssige Struktur, die mit der Hilfsenergie zusammengeführt werden. Im energielosen Zustand sind die Teile voneinander entfernt und haben keinen Kraftschluss. Mit Hilfsenergie werden die Teile zueinander bewegt und besitzen einen Formschluss, der die Blockade bewirkt (Fig.12).

Die Funktion kann aus Sicherheitsgründen umgedreht werden, sodass ohne Hilfsenergie die Blockade aktiviert wird und mit Hilfsenergie die Beweglichkeit gegeben ist.

Dieses Blockadesystem mit Hilfsenergie bietet die Möglichkeit, die Blockade zu beliebiger Zeit unabhängig von der Geschwindigkeit auszulösen. Die Auslösung kann von verschiedenartigen Sensoren wie Beschleunigungssensoren, Gyrometer oder Kraftsensoren ausgelöst werden. Sind beispielsweise an den Anbindungsstrukturen Beschleunigungs- sensoren angebracht, kann aus Differenzbildung und Integration die Auszugsgeschwindigkeit und über die Geometrie die Gelenksrotationsgeschwindigkeit ermittelt werden. Die gleiche Information erhält man aus der Differenz zweier Gyrometern, die beispielsweise an Ober- und Unterarm oder an Schulter und Hüfte montiert sind. Letzteres erfasst die Oberkörperrotation und somit die Wirbelsäulenausdrehung. Die Differenzbildung ist wichtig, da sich der Körper meist auch als Ganzes im Raum bewegt. Die Information über die Längenänderung zwischen den Anbindungsstrukturen kann auch vom Blockadeelement (1.1) selbst kommen, wenn ein integrierter Drehzahlsensor bzw. Drehpositionssensor wie in Fig.17 beschrieben eingesetzt wird.

Darüber hinaus können die Daten von diesen Sensoren mit anderen Parametern und Informationen verkoppelt werden, um die Auslösung der Blockade über einen Algorithmus einzuleiten. Überdies kann mit den aktuellen Daten der Sensoren prädiktiv eine Unfallsituation berechnet werden. Aus den verschiedenen Trajektorien von Körpersegmenten ist ersichtlich, ob ein Überschreiten der normalen physiologischen Auslenkungen von Körpersegmenten stattfinden wird.

Dazu ist auch das Wissen über das Fahrverhalten von Protektorträgern hilfreich, welches ebenfalls mit der beschriebenen Sensorik erfasst werden kann. Diese Information kann aus dem aktuellen Fahrstil oder auch aus den abgespeicherten früheren Fahrten gewonnen werden. Damit kann auch ein schlechter bzw. unsicherer Fahrer identifiziert werden, wodurch die Ansprechschwelle der Blockade reduziert werden kann und umgekehrt. In den Auslösealgorithmen können weitere Information einfließen, die von einem komplexen Informationsnetzwerk, beispielsweise mit anderen Fahrern und Trainern oder auch vom GPS kommen.

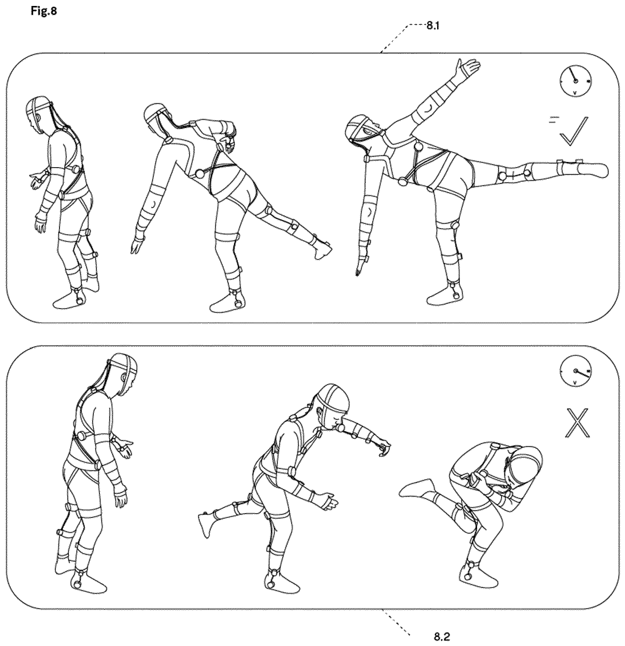

Werden mehrere Blockadeeinheiten an mehreren Körpersegmenten eingesetzt, so kann prozessorgesteuert eine optimale Blockadeabfolge definiert werden, damit in der dynamischen Unfallsituation die unterschiedlichen Massenträgheitskomponenten des menschlichen Körpers berücksichtigt werden und der Unfall beispielsweise möglichst wirbelsäulenschonend abläuft (Fig.8).

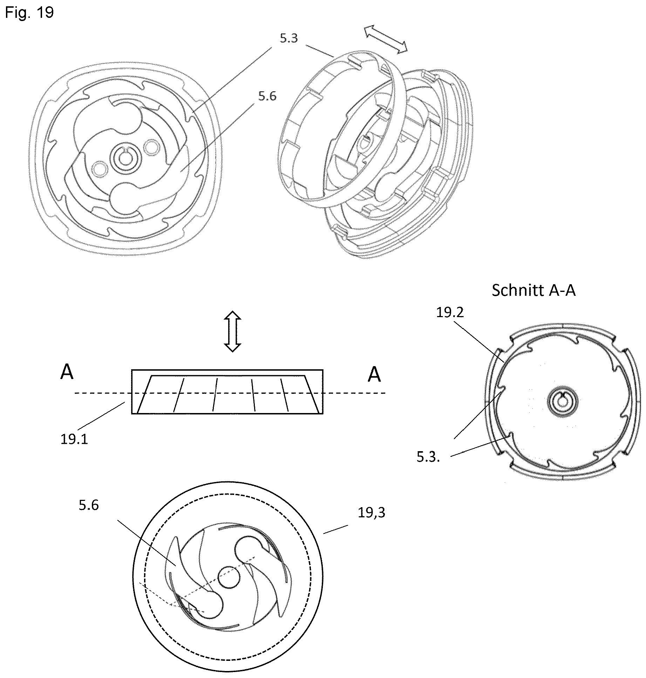

Eine Kombination zwischen selbstauslösendem und aktiven Blockadesystem kann erreicht werden, indem die Rotoranordnung mit Zentrifugalkraftprinzip des selbstauslösenden Systems eingesetzt wird, jedoch das formschlüssige Gegenstück im Stator mit Hilfsenergie verschiebbar ist, sodass der Formschluss wie beschrieben erfolgen oder nicht erfolgen kann (Fig.19). Dazu kann der Formschlussstruktur (5.3) in axialer Richtung verschoben werden. Ist diese ganz ausgefahren, können die Kontaktelemente (5.6) zwar durch die Rotation nach Außen geschleudert werden, aber keinen Formschluss erreichen und somit keine Blockade auslösen. Erst wenn die Formschlussstruktur (5.3) in die Struktur eingefahren wird, erfolgt die vorher beschriebene Blockade. Die Formschlussstruktur kann auch eine konische Innenstruktur (19.1) besitzen, wodurch je nachdem wie weit die der Formschlussstruktur eingefahren wird, der Luftspalt und somit der Auslenkweg des Blockadeelementes (5.6) und somit die Blockadeauslösung variiert. Somit kann die Auslösegeschwindigkeit mit geringer Hilfsenergie eingestellt werden. Die axiale Verschiebung kann beispielsweise derart erfolgen, dass die Formschußstruktur (5.3) auf einem Feingewinde am Gehäuse sitzt und über ein Getriebe oder Riementrieb von einem Schrittmotor gedreht wird und somit in der axialen Richtung verschiebbar ist. Diese Justage kann auch händisch erfolgen. Somit wäre die Auslösung für eine Person oder Anwendungsfall einstellbar aber fix.

### Figurenbeschreibung:

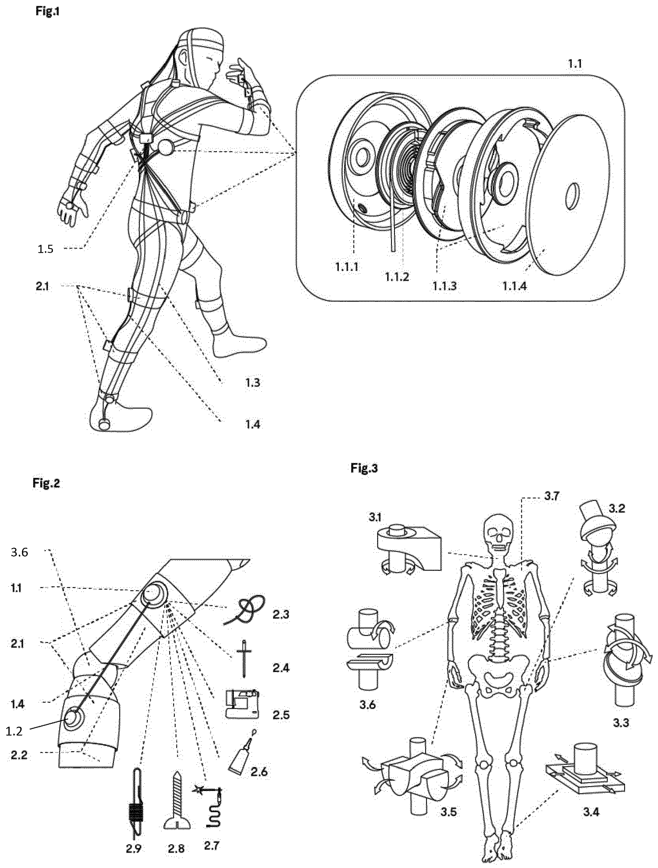

Fig. 1 zeigt eine Person in Bewegung, welche mit dem Blockadesystem ausgestattet ist, das aus mehreren Blockadeeinheiten (1.0) besteht. Die Blockadeeinheiten (1.0) setzen sich aus Blockadeelementen (1.1) und Zugelementen (1.4) zusammen, die an den Körperanbindungsstrukturen (2.1) befestigt sind. Die Blockadeelemente (1.1) bestehen aus Gehäuse (1.1.1, 1.1.4), einem Aufspulmechanismus (1.1.2) und einem Blockademechanismus (1.1.3).

Die einzelnen Blockadeelemente (1.1) können durch Steuerleitungen (1.3) mit einem zentralen Steuer- und Auslösegerät (1.5) verbunden sein.

Fig. 2 zeigt zwei über ein Scharniergelenk (3.6) verbundene Körpersegmente (2.2), an die jeweils eine Körperanbindungsstruktur (2.1) angebracht ist und auf der jeweils ein Blockadeelement (1.1) angebracht ist und mittels eines Zugelementes (1.4) verbunden sind. Das Blockadeelement kann durch verknoten (2.3), nieten (2.4), nähen (2.5), kleben (2.6) verschweißen (2.7), schrauben (2.8) und per Draht (2.9) mit der Körperanbindungsstruktur (2.1) verbunden werden. Das Blockadeelement (1.1) kann auch nur einseitig von Gelenk eingesetzt werden, wobei das Zugseil über die Zuganbindung (1.2) mit der korrespondierenden Anbindungsstruktur (2.1) verbunden wird.

Fig. 3 stellt ein menschliches Skelett (3.7) mit verschiedenen Körpersegmenten (2.2) dar, die durch Drehgelenke (3.1), Kugelgelenke (3.2), Kondylengelenke (3.3), planare Gelenksverbindungen (3.4), Sattelgelenke (3.5), Scharniergelenke (3.6) verbunden sind.

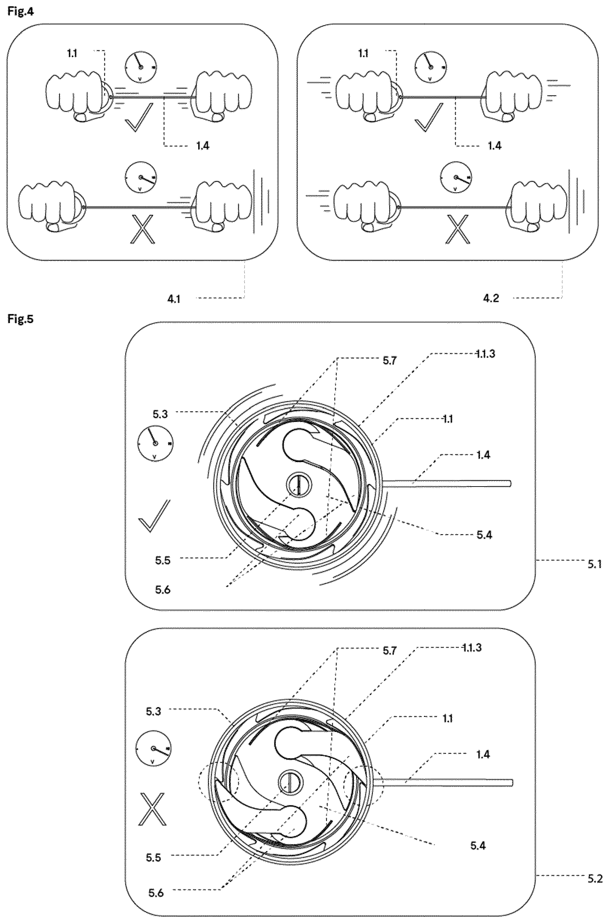

Fig. 4 zeigt zwei Hände, die jeweils ein Blockadeelement (1.1) und das andere Ende einer Zugelement (1.4) auseinanderziehen (4.1) oder zusammendrücken (4.2). Hier sieht man, dass das Blockadeelement unter einem definierten Geschwindigkeitsschwellwert frei beweglich ist, jedoch über einem definierten Geschwindigkeitsschwellwert blockiert.

Fig. 5 zeigt einen Blockademechanismus (1.1.3) eines Blockadeelementes (1.1) von oben, das durch das Zugelement (1.4) betätigt wird. Detail (5.1) zeigt den Zustand unter einem definierten Geschwindigkeitschwellwert, wo die Rückholfeder (5.7) stärker ist als die Zentrifugalkraft auf die Kontaktelementen (5.6) und somit der Blockademechanismus (1.1.3) frei beweglich ist. Die gelagerte Aufnahmescheibe (5.4) kann sich mit den darin liegenden federrückgestellten Kontaktelementen (5.6) um die Achse (5.5) frei bewegen.

Detail (5.2) zeigt den Zustand nachdem ein definierter Geschwindigkeitschwellwert überschritten wurde, indem der Blockademechanismus (1.1.3) durch den umliegende Formschlussstruktur (5.3) formschlüssig blockiert wurde. Das Auszugelement (1.4) wird beim bzw nach dem Eintritt in das Blockadelement (1.1) entweder in die tangentiale Richtung des Aufspulmechanismus (1.1.2) umgelenkt.

Die Richtung des Auszugselemente (1.4) wird zum Zentrum geführt und wird am Gehäuse in tangentiale Richtung zum Aufspulmechanismus (1.1.2) umgelenkt. Dadurch entsteht während der Blockade kein Drehmoment zwischen Gehäuse und Anbindungsstruktur (2.1). Wird dieses in Kauf genommen, kann das Auszugselement (1.4) auch tangential zum Aufspulmechanismus (1.1.2) das Gehäuse außermittig verlassen.

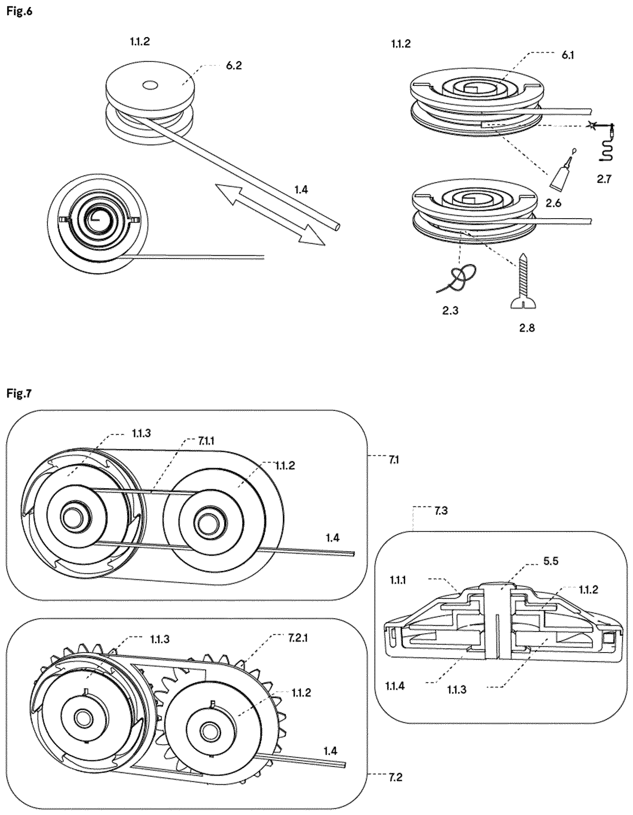

Fig. 6 zeigt einen rückstellenden Aufspulmechanismus, der durch einen Energiespeicher, wie beispielsweise eine Spiralfeder (6.1) betrieben wird. Das Zugelement (1.4) ist dadurch variabel ausziehbar und kann thermisch (z.B. verschwei-βen) (2.7), mechanisch (z.B. verknoten) (2.3), durch Verschrauben (2.8) oder chemisch (z.B. kleben) mit der Spule verbunden werden.

Fig. 7 zeigt drei Varianten der Blockadeelemente (1.1) in welche der Aufspulmechanismus (1.1.2), sowie der Blockademechanismus (1.1.3) radial und axial kraftübertragend zueinander angeordnet sind. Detail 7.1 zeigt eine radiale Anordnung des Aufspul- mechanismus (1.1.2) und des Blockademechanismus (1.1.3), welcher mit einem Riemen (7.1.1) kraftschlüssig verbunden sind.

Detail 7.2 zeigt eine radiale Anordnung des Aufspul- und Blockademechanismus (1.1.3), 1.1.2), welche mit einer Verzahnung (7.2.1) kraftschlüssig miteinander verbunden sind.

(7.3) zeigt einen Schnitt durch die Blockadeeinheit (1.1) in der der Aufspulmechanismus (1.1.2) und der Blockademechanismus (1.1.3) mittels einer Achse (5.5) kraftschlüssig verbunden sind und mit einem Gehäuse (1.1.1 / 1.1.4) umhüllt sind.

Detail 7.1 und Detail 7.2 haben den Vorteil, dass die Anordnung flacher wird.

Fig. 8 zeigt den Bewegungsablauf von Unfällen in zwei dreiteiligen Sequenzen.

Detail 8.1 zeigt die uneingeschränkte Bewegungsfreiheit bei einer definierten Geschwindigkeit. Detail 8.2 zeigt einen Unfall in dem die Blockadeeinheiten (1.1) blockieren und somit die Gelenke vor zu hoher physiologischer Belastung schützen.

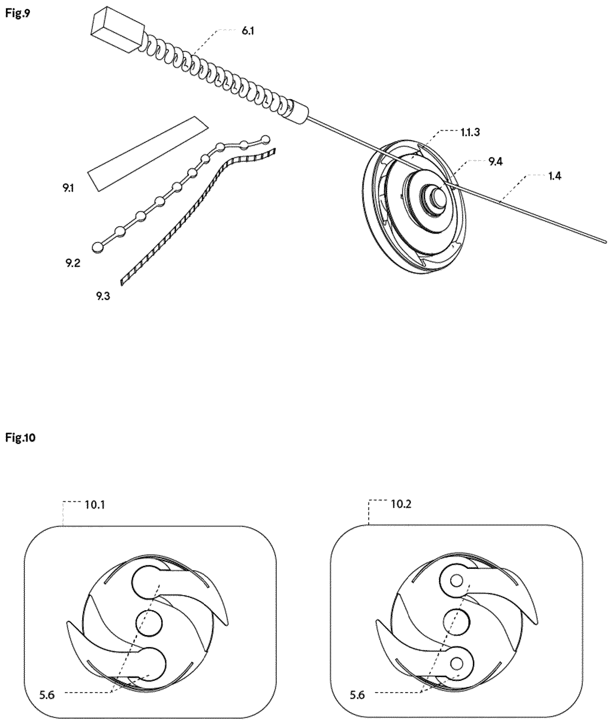

Fig. 9 zeigt eine Ausführungsvariante, in der der Energiespeicher (6.1) zur Straffung des Auszugelementes (4.1) und zum Wiederaufwickeln auf den Spulenkörper (6.2) extern angebracht wird. Die Kraftübertragung erfolgt über einen Umlenkmechanismus (9.4), der am Blockadeelement (1.1.3) angebracht ist. Das Zugelement (1.4) kann als Seil (1.4), Ketten (9.2), Riemen (9.3) oder Gurt (9.1) ausgeführt sein.

Fig. 10 zeigt zwei Ausführungsvarianten, in denen die Kontaktelemente (5.6) über einen konzentrischen Formschluss (10.1) oder über einen Lagerungsbolzen (10.2) eingebettet werden können. In beiden Abbildungen handelt es sich um eine zylindrische Lagerung mit radialer und axialer Fixierung, mit durch Anschläge begrenzter Rotationsfreiheit. Eingezeichnet sind auch die gekrümmten Blattfedern (5.7), die gegen die Zentrifugalkraft wirkt und die Kontaktelemente (5.6) unter einer bestimmten Rotationsgeschwindigkeit zentripedal zurückholen.

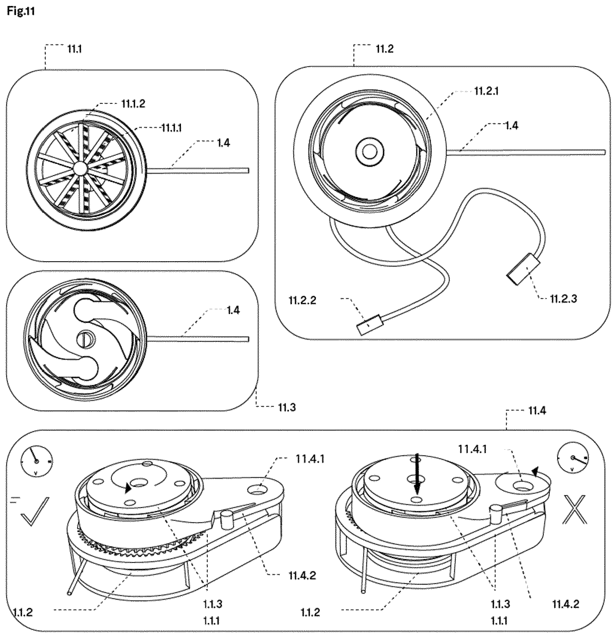

Fig. 11 zeigt drei Ausführungsvarianten des Blockademechanismus (1.1.3). Detail (11.1) zeigt den Blockaderotor (11.1.1) in einer Anordnung mit Flügelstruktur in einer vorzugsweise viskösen Flüssigkeit (11.1.2) eingebettet. Diese bieten bei kleiner Geschwindigkeit einen geringeren Widerstand als bei hoher. Überdies kann bei abrupt steigender Geschwindigkeit die Flüssigkeit (11.1.2) nicht schnell genug umgelenkt werden und der Rotor des Blockademechanismus (1.1.3) wird somit stärker blockiert. Der Effekt wird verstärkt, wenn die Rotorflügel beispielsweise stegartig (11.1.1) aufgebaut sind oder radiale Schlitze vorweisen in die starre, rechenförmige Lamellen greifen, die am Stator befestigt sind.

Detail (11.2) zeigt eine elektromagnetische Kupplung mit jeweils einem DrehzahlSensor (11.2.2, 11.2.3), sowie einer elektromagnetischen Spule (11.2.1), die bei definierter Drehzahl den Blockademechanismus (1.1.3) magnetisch arretiert.

Detail 11.3 zeigt einen Blockademechanismus (1.1.3) der durch Fliehkraft die Kontaktelemente (5.6) arretieren lässt.

Detail (11.4) zeigt ein Ausführungsbeispiel des Blockadeelements (1.1), indem der Blockademechanismus (1.1.3) mit dem Aufspulmechanismus (1.1.2) axial gelagert ist und als Einheit über einen definierten Drehpunkt (11.4.1) gelagert ist. Diese Einheit wird über eine Feder- Rückstellung (11.4.2) unter einem Belastungsschwellwert in einer definierten Position gehalten. Übersteigt die Energie den Belastungsschwellwert, so wird die Blockadeeinheit mit dem Aufstellmechanismus über den definierten Drehpunkt gegen das Gehäuse (1.1.1) formschlüssig gedrückt und somit arretiert.

Fig.12 zeigt beispielhaft zwei Ausführungen einer elektromagnetisch gesteuerten Blockadeeinheit. In Detail 12.1, bzw. in der Vergrößerung in Detail 12.3 sieht man eine Variante mit zwei axial angeordneten Arretierungsplatten (12.4), (12.5), wobei (12.5) auf der Achse verschiebbar ist, aber ein Drehmoment beispielsweise über Nut und Feder auf die Achse übertragen kann. (12.4) ist starr mit dem Stator verbunden. Ohne elektromagnetische Aktivierung werden die beiden Platten durch eine Feder (12.6) auseinander gehalten.

12.3. zeigt eine Detailansicht vom (12.1) und (12.2) aus einem andern Winkel mit den beiden Arretierungsplatten (12.4), (12.5), welche für den Formschluss komplementären Profilen aufweisen.

Die Blockade wird dadurch erreicht, dass die untere Platte (12.5) in die obere Platte (12.4) formschlüssig eingreift, dadurch erreicht, dass durch eine stromdurchflossene elektrische Spule (12.2.1) ein Magnetfeld erzeugt wird, welches durch den Gehäuseteil (12.2.7), die feststehende Platte (12.4), die rotierende Platte (12.5) und das ringförmige Joch (12.8) geschlossen wird und im Luftspalt zwischen den Platten (12.4) und (12.5) Anziehungskräfte auf die Platten ausgeübt wird. Ohne ein formschlüssiges Profil ist auch eine Reibungskupplung realisierbar.

In Detail 12.2 sieht man ein weiteres Ausführungsbeispiel in dem das Gehäuse (1.1.4, 1.1.1) die elektromagnetische Spule (11.2.1) umschließt.

Fig.13 zeigt zwei Körperanbindungsstrukturen (2.1) in denen die Blockadeeinheit (1.1) mit einem Zugelement (1.4) durch einen Umlenkmechanismus (13.1, 13.2) mit sich selbst gekoppelt wird. Das heißt, eine Seite vom Zugelement (1.4) führt wie bisher beschrieben zur Trommel des Blockadesystems, wobei das andere Ende von (1.4) über die Umlenkung (13.1 bzw. 13.2) zu einem fixen Punkt beispielsweise am Gehäuse der Blockadeeinheit oder zur Körperanbindung (2.1) selbst führt. Somit wird die Maximalbelastung infolge des Flaschenzugprinzips verdoppelt. Dies wird durch Gleitflächen (13.1) oder durch gelagerte zylindrische Körper (13.2) erreicht.

Gleichzeitig mit der Verdoppelung der Blockadekraft wird die Auszuglänge des Seiles aus der Spule verdoppelt, was bei gleicher Zeiteinheit zu einer Verdoppelung der Rotationsgeschwindigkeit führt und folgedessen die Fliehkräfte beziehungsweise visköse Effekte und somit die Blockier- beziehungsweise Bremsfunktion erhöht.

Fig.14 stellt ein abstrahiertes Scharnieergelenk des menschlichen Körpers dar, in dem man sieht, dass eine Biegung in der neutralen Faserlinie (14.2) keinen Längenversatz bewirkt. Versetzt man nun parallel zur neutralen Faserlinie (14.2) die Versatzflächen (14.3), so wird die Längenveränderung mit dem Abstand vom Drehpunkt signifikant.

Fig. 15 zeigt eine weitere Variante eines Blockadesystems (1.1), welche zwei Zugelemente (1.4a, 1.4.b) besitzt, welche aus zwei gegenüberliegenden Auslässen in entgegengesetzte Richtung entlang der Kraftlinie zu den beiden Körperanbindungsstrukturen (2.1) führen. Die beiden Zugelement (1.4a, 1.4.b) sind auf dem Spulenkörper (5.4) im gleichen Richtungssinn aufgewickelt und können übereinander (15.4) oder in zwei separaten Wickelkammern (15.3) nebeneinander liegen. Der Vorteil liegt darin, dass das Blockadesystems (1.1) selbst keine Anbindung (vernähen, verkleben, vernieten etc.) zu einer Körperanbindungsstuktur (2.1) haben muss und das Gehäuse kleiner gestaltet werden kann. Oft ist auch bei der Körperanbindungsstuktur (2.1.) wenig Platz vorhanden, sodass ein Blockadesystems (1.1) dort stören würde. Bei Verwendung zweier Wickelkammern (15.3) auf dem Spulenkörper, können diese unterschiedliche Durchmesser (15.5) besitzen, wodurch sich die Position des Blockadesystems (1.1) nicht symmetrisch zwischen den Aufhängepunkten einstellt, sondern sich mehr zu einem Befestigungspunkt bewegt, wodurch auf unterschiedliche Gegebenheiten von Gelenktypen und das Designe von Kleidungsstücken Rücksicht genommen werden kann.

(15.a) zeigt im Aufriss das Grundprinzip der zweifachen Wicklung, wobei (15.b.) das Prinzip einer praktischen Ausführung zeigt, welches im Gehäuse zwei Bolzen (15.1) enthält, welche einerseits die Zugelemente (4.1a, 4.1b) in die Tangente (15.1) des Aufspulmechanismus (1.1.2) umlenken und gleichzeitig zumindest ein Bolzen die Verankerung für die Spiralfeder darstellen. 15.c und 15.d zeigen Spulenvarianten.

Fig.16 zeigt die Verwendung von Reflexlichtsensoren (16.1), die im Gehäuse (16.7) implementiert sind und die detektieren, wenn die Kontaktelemente (5.6), (16.6) während der Rotation den Sensor passieren. In Position (16.a) befindet sich ein Kontaktelemente (5.6) (16.6) vor dem Sensor, wodurch das ausgestrahlte Licht (16.2) stark reflektiert wird (16.3a). In Position (16.b) ist die Distanz größer und das ausgestrahlte Licht (16.2) wird schwächer reflektiert (16.3b). Es kann ein Sensor eingesetzt werden oder auch mehrere. Dargestellt sind hier zwei Sensoren. Damit wird es möglich, auch die Rotationsrichtung zu detektieren, wenn die beiden Sensoren <90° beispielsweise 45° positioniert werden oder beide Hacken eine unterschiedliche hell-dunkel Oberflächencodierung (16.4) und (16.5) besitzen. Vorzugsweise wird für die Unterscheidung "Hacken-kein Hacken" eine Schwelle definiert und das Signal digital weiterverarbeitet (Zeitmessung).

Die Rotationsrichtung kann auch mit nur einem Sensor gemessen werden, wenn das reflektierte Signal analog aufgenommen wird. Aufgrund der Form des Kontaktelemente (5.6) (16.6) schaut die gemessene Kurve des Reflexlichtes nämlich drehrichtungsabhängig aus.

Anstatt eines Reflexlichtsensors (16.1) kann auch ein magnetischer Sensor in Form einer Detektorspule oder eines Hall-Sensors, oder ein Ultraschallsensor oder eine Mikrophonkapsel eingesetzt werden.

Fig. 17 zeigt eine Möglichkeit, wie aus der Bewegung von Körpersegmenten zueinander unter Verwendung eines Blockadesystems elektrische Energie gewonnen werden kann. Dazu wird im rotierenden Teil (17.8) am Umfang oder an einer der Stirnseite nahe am Umfang ein Permanentmagnet (17.2) positioniert, welcher über sein Magnetfeld (17.3) in einer feststehenden Spule (17.1) eine Spannung induziert. Die Ausrichtung des Magneten kann wie in Detail 17.b dargestellt in radialer Richtung oder in tangentialer Richtung (17.c) erfolgen. Zur Verstärkung des magnetischen Flusses kann ein Spulenkern (17.4) beispielsweise aus Eisen oder Ferrit verwendet werden. Wird die Spule nicht direkt in das Gehäuse eingebaut, kann die Spule (17.1a) auch außerhalb des Gehäuses positioniert werden und der magnetische Fluss wird mit einem längeren Eisenkern (17.5) nach außen geleitet. Die bremsende Wirkung während des Rückspulens des Auszugelements kann verhindert werden, wenn mittels Elektronik in dieser Phase die Last abgetrennt wird.

Fig.18 zeigt die Möglichkeit, die Blattfeder (5.7) mittels eines Exzenters (18.1) variabel vorzuspannen und somit die Rückstellkraft einzustellen.

Fig. 19 zeigt die Formschlussstruktur (5.3), die in axialer Richtung ausfahren kann und somit die Blockadefunktion deaktiviert werden kann. Eingezeichnet ist der eingefahrene Zustand, der die Blockade ermöglicht.

(19.1) zeigt eine konische Formschlussstruktur (5.3) im Aufriss, (19.2) zeigt diese Struktur durch den Schnitt AA (19.1) im Grundriss und (19.3) zeigt den Rotor mit den Kontaktelementen (5.6) im Grundriss. Wird die Formschlussstruktur (19.1) in axialer Richtung verschoben, so ändert sich der Luftspalt und die Kontaktelemente (5.6) müssen verschieden weit ausfahren, wodurch die dazu notwendige Drehzahl unterschiedlich ist.

(5.3) kann auch ohne formschlüssige Struktur ausgelegt sein (auch für (19.1)), wodurch wie bei einer Trommelbremse die Rotation gebremst wird.

## Patentansprüche

1. Blockadesystem zur Begrenzung der physiologischen Bewegungen von Körpersegmenten, wobei Körpersegmente mittels eines Auszugelementes (1.4) und eines Blockadeelementes (1.1) miteinander verbunden sind, derart, dass das Auszugelement (1.4) auf einem Aufspulmechanismus (1.1.2) im Blockadeelement (1.1) aufgewickelt ist, beim Ausziehen einen gekoppelten Blockademechanismus (1.1.3) antreibt, welcher bei Überschreitung einer bestimmten Geschwindigkeit eine weiter Drehung ganz oder teilweise blockiert.

2. Blockadesystem nach Anspruch 1 **dadurch gekennzeichnet, dass** dieses zumindest aus einer Blockade Einheit (1.0, 1.1) besteht, welche am Körper über mindestens zwei festgelegte Verbindungspunkte an variabel miteinander verbundenen Körpersegmenten getragen wird.

3. Blockadesystem nach Anspruch 1 und 2 **dadurch gekennzeichnet, dass** es unter einem Geschwindigkeitsschwellwert beweglich ist, und/oder dass dieses eine Blockade durch einen Blockademechanismus (1.1.3) über einem Geschwindigkeitsschwellwert einleitet.

4. Blockadesystem nach Anspruch 1 bis 3 **dadurch gekennzeichnet, dass** es einen sich rückstellenden Aufspulmechanismus (1.1.2) beinhaltet, der eine Längenveränderung in einem spezifischen Bereich ermöglicht, welcher kraftübertragend an einen Blockademechanismus (1.1.3) gekoppelt ist, der formschlüssig oder über Reibung den Aufspulmechanismus (1.1.2) blockiert und die Längenveränderung verhindert und somit die auftretende Kraft aufnimmt.

5. Blockadesystem nach einem oder mehreren der vorhergehenden und nachfolgenden Ansprüchen **dadurch gekennzeichnet, dass** verschiedene Körpersegmente, die über verschiedenen Gelenken wie Drehgelenke, Scharniergelenke, Sattelgelenke, planaren Gelenksverbindungen, Kondylen- oder Kugelgelenken einzeln oder in Kombination miteinander verbunden sind mit dem Blockadesystem ausgestatten werden können.

6. Blockadesystem nach einem oder mehreren der vorhergehenden und nachfolgenden Ansprüchen **dadurch gekennzeichnet, dass** die gegenüberliegenden Teile des Blockadesystems durch aufnähen, aufnieten, anknoten, kleben, verschweißen, schrauben, per Draht verdrahten, an der Körperanbindungsstruktur verbunden werden können.

7. Blockadesystem nach einem oder mehreren der vorhergehenden und nachfolgenden Ansprüchen **dadurch gekennzeichnet, dass** die Längenveränderung durch eine Spule oder Rolle realisiert wird, auf der ein Zugelement in Form einer Schnur, Draht, Seil, Riemen, Band oder Kette aufgerollt oder umgelenkt wird.

8. Blockadesystem nach einem oder mehreren der vorhergehenden und nachfolgenden Ansprüchen **dadurch gekennzeichnet, dass** der sich rückstellende Aufspulmechanismus durch einen Energiespeicher betrieben wird, der sich im linearen Spannungsbereich befindet.

9. Blockadesystem nach einem oder mehreren der vorhergehenden und nachfolgenden Ansprüchen **dadurch gekennzeichnet, dass** es eine mechanische Kupplung in Form einer gelagerten Aufnahmeplatte in der mindestens ein Kontaktelement (5.6) drehbar gelagert ist, beinhaltet.

10. Blockadesystem nach einem oder mehreren der vorhergehenden und nachfolgenden Ansprüchen **dadurch gekennzeichnet, dass** die Kontaktelemente (5.6) durch Stifte oder einen Formschluss gelagert werden, und/oder dass die Kontaktelemente (5.6) bei Überschreitung des Schwellwerts eine Bindefläche mittels Formschluss mit dem umliegenden Gehäuse bilden und somit eine Blockade erwirken.

11. Blockadesystem nach einem oder mehreren der vorhergehenden und nachfolgenden Ansprüchen **dadurch gekennzeichnet, dass** der Blockademechanismus (1.1.3) abhängig vom Schwellwert mechanisch, elektrisch, elektromagnetisch, viskos, durch Fliehkraft oder einer Kombination derer auslöst.

12. Blockadesystem nach einem oder mehreren der vorhergehenden und nachfolgenden Ansprüchen **dadurch gekennzeichnet, dass** die Blockadeeinheiten (1.0, 1.1) entweder mechanisch, elektronisch, oder aus einer Kombination derer ausgelöst werden können, sowie in einem Blockadesystem in welchem die Blockadeeinheiten (1.0, 1.1) drahtlos oder mittels Steuerleitungen mit einem zentralen Steuer- und Auslösegerät (1.5) verbunden sind.

13. Blockadesystem nach einem oder mehreren der Ansprüche 1-12 **dadurch gekennzeichnet, dass** auf dem Spulenkörper (6.2) übereinander in einer Wickelkammer (15.4) oder in zwei getrennten Wickelkammern (15.3) zwei Zugelement (1.4a, 1.4.b) gleichsinnig aufgewickelt sind, derart, dass diese zwei Zugselemente in entgegengesetzter Richtung den Spulenkörper (6.2) verlassen und zu den Körperanbindungsstrukturen (2.1) führen, wodurch sich das Blockadeelemente (1.1) zwischen den Körperanbindungsstrukturen (2.1) positioniert.

14. Blockadesystem nach Anspruch 1 und 13 **dadurch gekennzeichnet, dass** die zwei getrennten Wickelkammern (15.3) des Spulenkörper (6.2) unterschiedliche Durchmesser haben können, wodurch eine asymmetrische Auszugslänge entsteht.

15. Blockadesystem nach einem oder mehreren der Ansprüche 1-14 **dadurch gekennzeichnet, dass** zur Messung der Umdrehungen des Rotors (5.4) des Blockadeelementes (1.1) ein oder mehrere Sensoren eingesetzt werden, die die Position des Kontaktelementes (5.6) entweder optisch mit Reflexlichtsensoren, magnetisch mit einer Detektorspule oder einem Hallsensor oder mit einem Ultraschallsensor messen und im Statorgehäuse am Umfang der Innenseite zugewandt oder seitlich in der Höhe der Innenseite zugewandt positioniert werden.

16. Blockadesystem nach einem oder mehreren der Ansprüche 1-15 **dadurch gekennzeichnet, dass** die Rotation zur Energiegewinnung verwendet wird, indem im rotierenden Teil (17.8) am Umfang oder an einer der Stirnseite nahe am Umfang ein Permanentmagent (17.2) positioniert wird, dessen bewegtes Magnetfeld (17.3) in einer Spule (17.1) im Stator eine Spannung induziert.

## Claims

1. A blocking system for limiting the physiological movements of body segments, wherein body segments are connected to one another by means of an extending element (1.4) and a blocking element (1.1) such that the extending element (1.4) is wound up on a winding mechanism (1.1.2) in the blocking element (1.1), and drives a coupled blocking mechanism (1.1.3) upon extension, which completely or partially blocks a further rotation when a specific speed is exceeded.

2. The blocking system according to Claim 1, **characterised in that** said blocking system consists of at least one blocking unit (1.0, 1.1) which is worn on the body by way of at least two fixed connection points on body segments which are variably connected to one another.

3. The blocking system according to Claim 1 and 2, **characterised in that** it is movable below a speed threshold value and/or **in that** said blocking system initiates a blockage by a blocking mechanism (1.1.3) above a speed threshold value.

4. The blocking system according to Claim 1 to 3, **characterised in that** it comprises a resetting winding mechanism (1.1.2) which makes possible a change in length in a specific range, which is coupled to a blocking mechanism (1.1.3) to transmit a force which blocks the winding mechanism (1.1.2) positively or by way of friction and prevents the change in length and consequently absorbs the force which occurs.

5. The blocking system according to one or more of the preceding and following claims, **characterised in that** various body segments which are connected to one another individually or in combination via various joints such as swivel joints, hinge joints, saddle joints, planar joint connections, condylar or ball joints can be equipped with the blocking system.

6. The blocking system according to one or more of the preceding and following claims, **characterised in that** the parts of the blocking system lying opposite one another can be connected to the body attachment structure by sewing, riveting, tying, gluing, welding, screwing, or wiring.

7. The blocking system according to one or more of the preceding and following claims, **characterised in that** the change in length is realised by a coil or a reel on which a tension element in the form of a cord, a wire, a cable, a strap, a belt or a chain is rolled up or looped around.

8. The blocking system according to one or more of the preceding and following claims, **characterised in that** the resetting winding mechanism is powered by an energy store which is located in the linear voltage range.

9. The blocking system according to one or more of the preceding and following claims, **characterised in that** it comprises a mechanical coupling in the form of a mounted receiving disk in which at least one contact element (5.6) is rotatably mounted.

10. The blocking system according to one or more of the preceding and following claims, **characterised in that** the contact elements (5.6) are mounted by pins or positively, and/or
**in that** the contact elements (5.6) form a bonding surface by means of a positive connection with the surrounding housing when the threshold value is exceeded and consequently effect a blockage.

11. The blocking system according to one or more of the preceding and following claims, **characterised in that** the blocking mechanism (1.1.3) triggers mechanically, electrically, electromagnetically, viscously, by centrifugal force or a combination thereof depending on the threshold value.

12. The blocking system according to one or more of the preceding and following claims, **characterised in that** the blocking units (1.0, 1.1) can either be triggered mechanically, electronically or by a combination thereof, as well as in a blocking system in which the blocking units (1.0, 1.1) are connected wirelessly or by means of control lines to a central control and triggering device (1.5).

13. The blocking system according to one or more of Claims 1-12, **characterised in that** two tension element (1.4a, 1.4b) are wound up on the coil body (6.2) above one another in one winding chamber (15.4) or in two separate winding chambers (15.3) in the same direction such that these two tension elements exit the coil body (6.2) in an opposite direction and lead to the body attachment structures (2.1), as a result of which the blocking elements (1.1) is positioned between the body attachment structures (2.1).

14. The blocking system according to Claim 1 and 13, **characterised in that** the two separate winding chambers (15.3) of the coil body (6.2) can have different diameters, as a result of which an asymmetrical extension length results.

15. The blocking system according to one or more of Claims 1-14, **characterised in that** one or more sensors are utilised to measure the revolutions of the rotor (5.4) of the blocking element (1.1), which measure the position of the contact element (5.6) either optically with reflected light sensors, magnetically with a detector coil, or a Hall sensor or with an ultrasonic sensor, and are positioned in the stator housing on the circumference facing the interior or laterally in height facing the interior.

16. The blocking system according to one or more of Claims 1-15, **characterised in that** the rotation is used to generate energy by a permanent magnet (17.2) being positioned in the rotating part (17.8) on the circumference or on one of the end faces near the circumference, the moving magnetic field (17.3) of which permanent magnet induces a voltage in a coil (17.1) in the stator.

## Revendications

1. Système de blocage destiné à limiter les mouvements physiologiques de segments corporels, les segments corporels étant reliés l'un à l'autre au moyen d'un élément d'extraction (1.4) et d'un élément de blocage (1.1), de telle sorte que l'élément d'extraction (1.4) est enroulé sur un mécanisme d'embobinage (1.1.2) dans l'élément de blocage (1.1) et entraîne, lors de l'extraction, un mécanisme de blocage (1.1.3) accouplé, qui bloque totalement ou partiellement une rotation supplémentaire lors du dépassement d'une vitesse déterminée.

2. Système de blocage selon la revendication 1, **caractérisé en ce que** celui-ci est constitué par au moins une unité de blocage (1.0, 1.1) qui est portée sur le corps par l'intermédiaire d'au moins deux points de liaison fixes sur des segments corporels reliés l'un à l'autre de manière variable.

3. Système de blocage selon la revendication 1 et 2, **caractérisé en ce qu'**il est mobile sous une valeur seuil de vitesse et/ou **en ce que** celui-ci initie un blocage par un mécanisme de blocage (1.1.3) au-dessus d'une valeur seuil de vitesse.

4. Système de blocage selon la revendication 1 à 3, **caractérisé en ce qu'**il comporte un mécanisme d'embobinage (1.1.2) à rappel, qui permet une modification de longueur dans une plage spécifique, qui est accouplé avec transmission de force à un mécanisme de blocage (1.1.3) qui bloque, par complémentarité de forme ou par frottement, le mécanisme d'embobinage (1.1.2) et qui empêche la modification de longueur et qui absorbe donc la force qui survient.

5. Système de blocage selon l'une ou plusieurs des revendications précédentes et suivantes, **caractérisé en ce que** divers segments corporels, qui sont reliés les uns aux autres, individuellement ou en combinaison, par l'intermédiaire de diverses articulations, telles qu'une articulation rotative, une articulation à charnière, une articulation en selle, des liaisons articulées planaires, des articulations à condyle ou à rotule, peuvent être équipés du système de blocage.

6. Système de blocage selon l'une ou plusieurs des revendications précédentes et suivantes, **caractérisé en ce que** les parties opposées du système de blocage peuvent être reliées par couture, agrafage, nouage, collage, soudage, vissage, assemblage par des fils, à la structure d'attache au corps.

7. Système de blocage selon l'une ou plusieurs des revendications précédentes et suivantes, **caractérisé en ce que** la modification de longueur est réalisée par une bobine ou un rouleau, sur laquelle/lequel est enroulé ou dévié un élément de traction sous la forme d'un cordon, d'un fil, d'une corde, d'une courroie, d'un ruban ou d'une chaîne.

8. Système de blocage selon l'une ou plusieurs des revendications précédentes et suivantes, **caractérisé en ce que** le mécanisme d'embobinage à rappel est entraîné par un accumulateur d'énergie qui se trouve dans la zone de tension linéaire.

9. Système de blocage selon l'une ou plusieurs des revendications précédentes et suivantes, **caractérisé en ce qu'**il comporte un accouplement mécanique sous la forme d'une plaque d'absorption supportée, dans laquelle au moins un élément de contact (5.6) est logé rotatif.

10. Système de blocage selon l'une ou plusieurs des revendications précédentes et suivantes, **caractérisé en ce que** les éléments de contact (5.6) sont logés par des broches ou une liaison de forme et/ou **en ce que** les éléments de contact (5.6), lors du dépassement de la valeur seuil, forment une surface de resserrage par liaison de forme avec le logement environnant et provoquent ainsi un blocage.

11. Système de blocage selon l'une ou plusieurs des revendications précédentes et suivantes, **caractérisé en ce que** le mécanisme de blocage (1.1.3) déclenche, en fonction de la valeur seuil, de manière mécanique, électrique, électromagnétique, visqueuse, par une force centrifuge ou une combinaison de celles-ci.

12. Système de blocage selon l'une ou plusieurs des revendications précédentes et suivantes, **caractérisé en ce que** les unités de blocage (1.0, 1.1) peuvent être déclenchées de manière soit mécanique, soit électronique, soit par une combinaison de celles-ci ainsi que dans une système de blocage, dans lequel les unités de blocages (1.0, 1.1) sont reliées sans fil ou au moyen de lignes de commande à un appareil (1.5) central de commande et de déclenchement.

13. Système de blocage selon l'une ou plusieurs des revendications 1-12, **caractérisé en ce que** deux éléments de traction (1.4a, 1.4.b) sont enroulés dans le même sens sur le corps de bobine (6.2), l'un au-dessus de l'autre dans une chambre d'enroulement (15.4) ou dans deux chambres d'enroulement (15.3) séparées, de telle sorte que ces deux éléments de traction quittent le corps de bobine (6.2) dans un sens opposé et vont jusqu'aux structures d'attache au corps (2.1), moyennant quoi l'élément de blocage (1.1) se positionne entre les structures d'attache au corps (2.1).

14. Système de blocage selon la revendication 1 et 13, **caractérisé en ce que** les deux chambres d'enroulement (15.3) séparées du corps de bobine (6.2) peuvent présenter des diamètres différents, moyennant quoi on obtient une longueur d'extraction asymétrique.

15. Système de blocage selon l'une ou plusieurs des revendications 1-14, **caractérisé en ce que**, pour la mesure des rotations du rotor (5.4) de l'élément de blocage (1.1), un ou plusieurs capteurs sont utilisés, qui mesurent la position de l'élément de contact (5.6) soit de manière optique à l'aide de capteurs de lumière réfléchie, soit de manière magnétique à l'aide d'une bobine de détection ou à l'aide d'un capteur à effet Hall, soit à l'aide d'un capteur à ultrasons, et qui sont positionnés dans le boîtier du stator à la périphérie de manière orientée vers l'intérieur ou latéralement dans la hauteur, de manière orientée vers l'intérieur.

16. Système de blocage selon l'une ou plusieurs des revendications 1-15, **caractérisé en ce que** la rotation est utilisée pour la production d'énergie **en ce qu'**un aimant permanent (17.2) est positionné dans la partie rotative (17.8) à la périphérie ou sur l'une des faces frontales à proximité de la périphérie, dont le champ magnétique (17.3) en mouvement induit une tension dans une bobine (17.1) dans le stator.
